# EUROPEAN PATENT APPLICATION

(11) **EP 4 437 974 A1**
(43) Date of publication of application: **02.10.2024**
(21) Application number: 23220655.7
(22) Date of filing: 28.12.2023
(51) Int. Cl.: A61B 17/00

(54) **LEFT ATRIAL APPENDAGE OCCLUDER DELIVERY SYSTEM**

(30) Priority: 30.03.2023 US 202363493282 P
(71) Applicant: St. Jude Medical, Cardiology Division, Inc., St. Paul, MN 55117 (US)
(72) Inventor: Perszyk, Brian Joseph, Shoreview, 55126 (US); Hagel, Christopher, Ramsey, 55303 (US); Elmquist, Christian, Mounds View, 55112 (US); Larson, Ellie, Hutchinson, 55350 (US)
(74) Representative: Gill Jennings & Every LLP

(57) **Abstract**

In some examples, a delivery device includes a handle, a catheter sheath defining a lumen and extending distally from the handle, and a hemostasis valve positioned at least partially within the handle, the hemostasis valve being located proximal the catheter sheath, the hemostasis valve including a first seal and a second seal, and a first flush-port disposed between the first seal and the second seal.

## Description

### Cross-Reference to Related Applications

This application claims priority to U.S. Serial No. 63/493,282, filed March 30, 2023, the content of which is hereby incorporated by reference in its entirety as if fully set forth herein.

### Background

Catheters are frequently used to assist in the delivery of medical devices into a patient non-invasively. For example, several types of collapsible and expandable medical devices may be delivered to, and implanted within, the heart of a patient using a catheter that is advanced through the vasculature and into the patient's heart without needing to make any incisions in the patient's chest or heart, and without needing to put the patient on cardiopulmonary bypass.

Left atrial appendage ("LAA") occluder devices are one example of collapsible and expandable medical devices that may be delivered to a patient's heart via a catheter that traverses the patient's vasculature. In some examples, a catheter may be advanced through the patient's femoral vein, into the right atrium through the inferior vena cava, across the atrial septum and into the left atrium, with a distal end of the catheter positioned within or adjacent to the LAA. The LAA occluder device may be within the catheter during the advancement of the catheter, or otherwise may be advanced through the catheter after the catheter is already in the desired position. The LAA occluder may be in a collapsed state with a relatively small profile while inside the catheter, and may self-expand into the LAA upon deployment from the distal end of the catheter. One such LAA occluder is the Amplatzer^{™} Amulet^{™} Occluder offered by Abbott Labs. One example of a LAA occluder device is described in U.S. Patent No. 10,201,337, the disclosure of which is hereby incorporated by reference herein.

As explained in greater detail below, although this disclosure generally focuses on a steerable sheath for delivering a LAA occluder, the disclosure is not so limited, and may apply to various other types of sheaths (including non-steerable sheaths) and various other types of medical devices to be delivered (including other occluder-type devices, such as PFO closure devices, and other devices that are not occluders).

### Brief Summary

In some examples, a delivery device includes a handle, a catheter sheath defining a lumen and extending distally from the handle, and a hemostasis valve positioned at least partially within the handle, the hemostasis valve being located proximal the catheter sheath, the hemostasis valve including a first seal and a second seal, and a first flush-port disposed between the first seal and the second seal.

In some examples, a delivery device includes a handle, a catheter sheath defining a lumen and extending distally from the handle, the catheter sheath having a deflectable proximal curve and a deflectable distal curve spaced from the deflectable proximal curve, at least one pull ring formed within the sheath, one or more pull wires coupled to the at least one pull ring, and a first deflection knob for actuating the catheter sheath at the deflectable proximal curve and a second deflection knob for actuating the catheter sheath at the deflectable distal curve.

### Brief Description of the Drawings

Fig. 1A is a schematic view of a steerable sheath according to one aspect of the disclosure.
Fig. 1B is a schematic representation of another example of a steerable sheath according to one aspect of the disclosure.
Fig. 1C is a schematic cross-sectional view of one example of a steerable sheath according to one aspect of the disclosure.
Figs. 1D-1M illustrate several examples of deflection of a steerable sheath.
Fig. 1N illustrates examples of different curvatures of a braided transseptal guiding introducer.
Fig. 2A is a schematic view of a dilator for use with the steerable sheath of Fig. 1A.
Figs. 2B-C are schematic cross-sectional views of two dilators with and without a step.
Fig. 2D is a schematic cross-sectional view of an integrated dilator centering feature.
Fig. 3A is a perspective view of a hemostasis valve assembly of the steerable sheath of Fig. 1A.
Fig. 3B is an exploded view of the hemostasis valve assembly of Fig. 3A.
Fig. 3C is a side view of the hemostasis valve assembly of Fig. 3A.
Fig. 3D is a cross-section of the hemostasis valve assembly of Fig. 3A taken along the section line 3D-3D of Fig. 3C.
Fig. 3E is an enlarged isolated view of portion 3E of Fig. 3D.
Figs. 3F is a top perspective view of a hemostasis valve of the hemostasis valve assembly of 3A.
Figs. 3G-H are side and top views, respectively, of the hemostasis valve of Fig. 3F illustrating slits forming the valve functionality.
Fig. 4A is perspective view of the hemostasis valve assembly of Fig. 3A assembled to a hemostasis valve knob.
Fig. 4B is an exploded view of the hemostasis valve assembly and the hemostasis valve knob of Fig. 4A.
Fig. 4C is a side view of the hemostasis valve assembly and knob of Fig. 4A.
Fig. 4D is a cross-section of the hemostasis valve assembly and knob of Fig. 4A taken along the section line 4D-4D of Fig. 4C.
Fig. 4E is a cross-section of the hemostasis valve assembly and knob of Fig. 4A taken along the section line 4E-4E of Fig. 4C, with certain components omitted from the view.
Fig. 4F is a cross-section of the hemostasis valve assembly and knob of Fig. 4A taken along the section line 4D-4D of Fig. 4C after activation of the valve bypass feature.
Fig. 4G is a schematic representation showing another example of a hemostasis valve assembly according to another embodiment.
Fig. 4H is an enlarged view of the hemostasis valve of Fig. 4G.
Fig. 4I-1 to 4I-3 are schematic representations showing examples of a Tuohy-Borst valve.

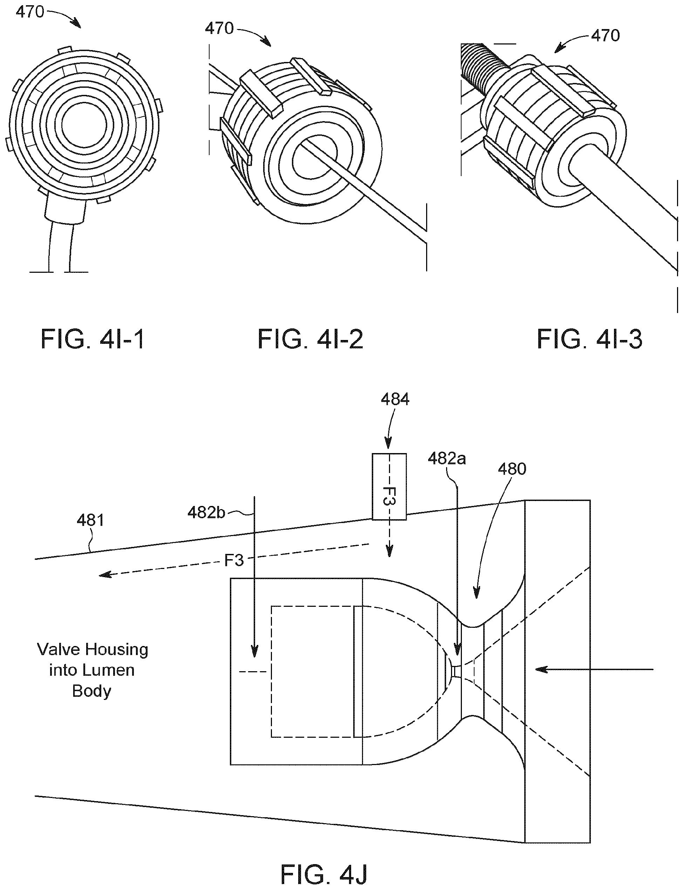

Fig. 4J is a schematic cross-sectional view of an hourglass hemostasis valve according to another embodiment.

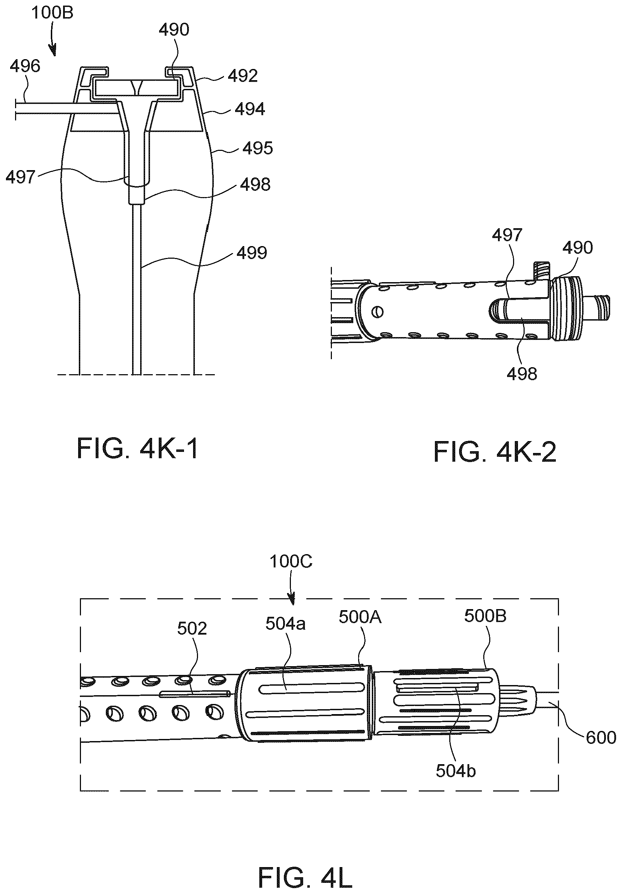

Fig. 4K-1 is a schematic cross-sectional view of the proximal end of a handle according to one embodiment.
Fig. 4K-2 is a schematic representation of a proximal end of a handle according to one embodiment.
Fig. 4L is a schematic representation showing a handle with deflection indicators according to one embodiment.

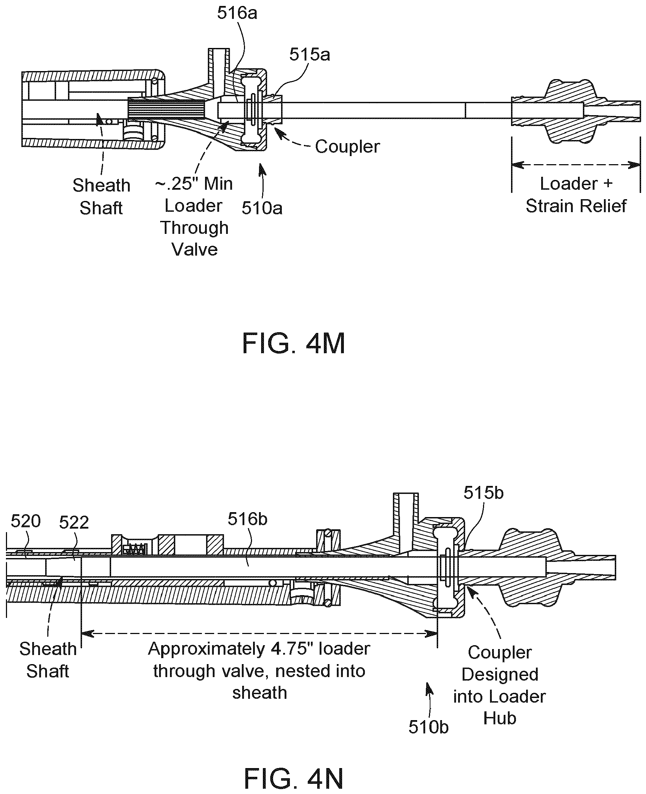

Figs. 4M-N are schematic cross-sectional drawings showing a loader assembly at least partially disposed within a handle.

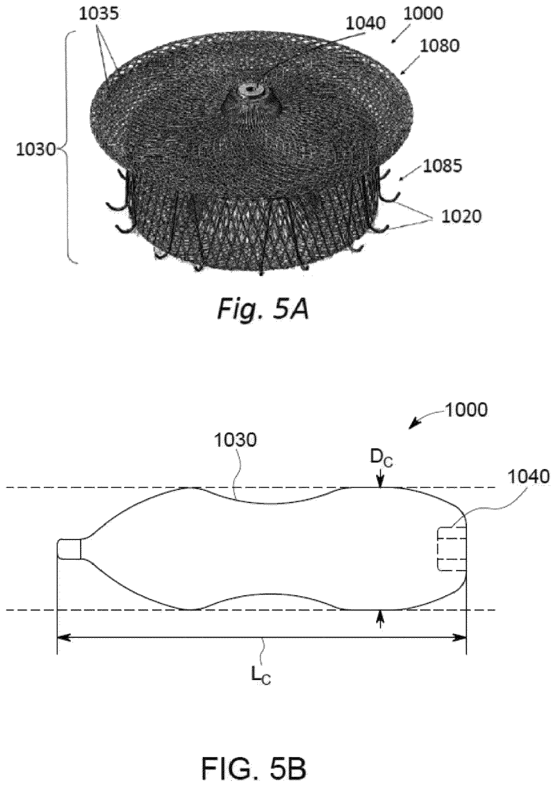

Fig. 5A is a perspective view of an occluder in an expanded or deployed condition.
Fig. 5B is a schematic view of the occluder of Fig. 5A in a collapsed or delivery condition.

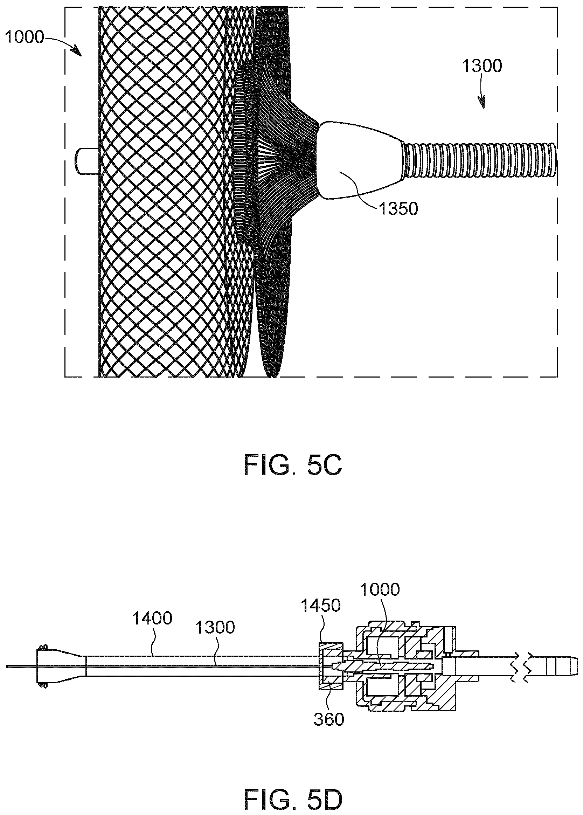

Fig. 5C is a side view of the occluder of Fig. 5A coupled to a delivery cable.

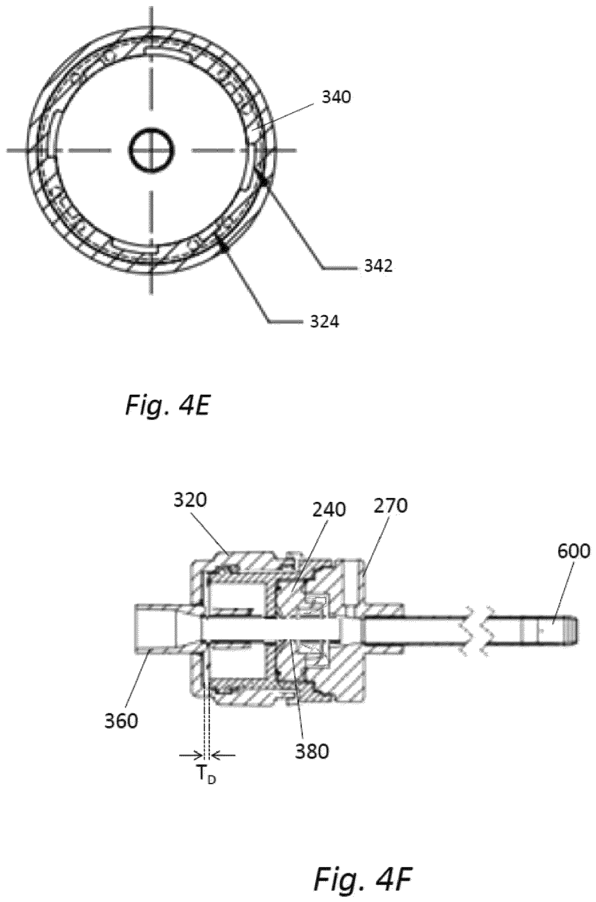

Fig. 5D is a highly schematic view of the occluder of Fig. 5A being passed through a loading tube coupled to the hemostasis valve knob and hemostasis valve assembly of Fig. 4F.

### Detailed Description

As used herein, the term proximal refers to a position relatively close to a user of a medical device, while the term distal refers to a position relatively far from the user of the medical device, when the medical device is being used in an intended manner. In other words, the leading end of a medical device is positioned distal to the trailing end of the medical device.

### Delivery Device

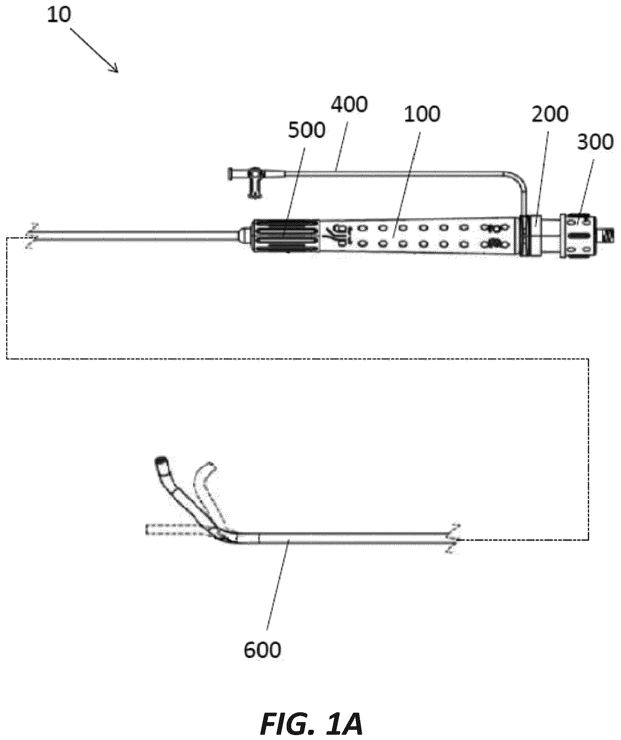

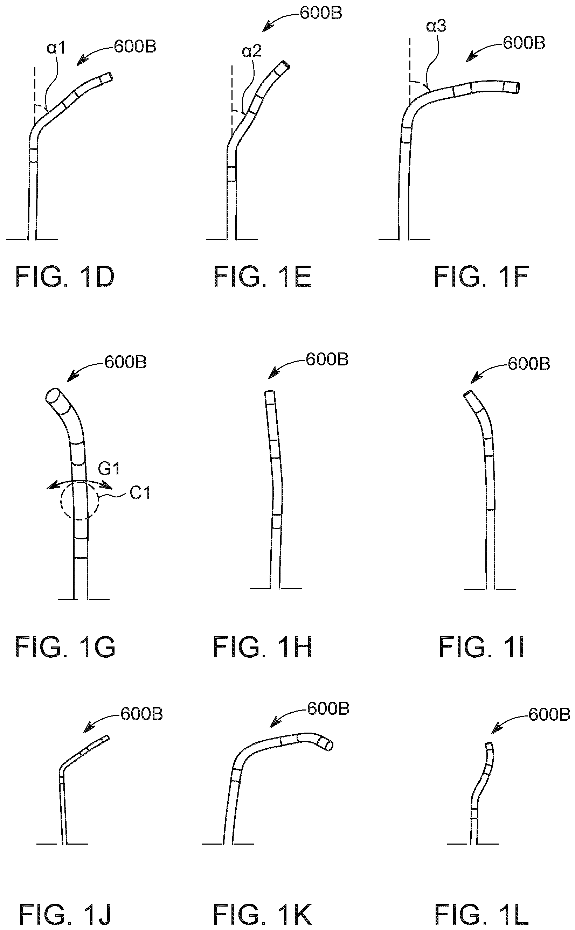

Fig. 1A illustrates a delivery device 10, which in the illustrated embodiment is a steerable sheath, although it should be understood that the inventive concepts disclosed herein may be used in conjunction with other catheter or sheath devices, whether or not steerable. Generally, delivery device 10 includes a handle 100, a hemostasis valve assembly 200 at a proximal end of the handle 100, a hemostasis valve knob (or actuator) 300, a flushing tube 400, a deflection knob 500 at a distal end of the handle 100, and a catheter or deflectable sheath 600 extending from a distal end of the deflection knob 500 to a terminal distal end of the delivery device 10. This disclosure includes improvements on various components of delivery device 10, and it will be understood that the features described may be combined in several permutations and that certain features are optional. Delivery device 10 is particularly suited for delivery of a collapsible and expandable LAA occluder, but it should be understood that the delivery device 10 may be suited to delivery of other medical devices.

The handle 100 may be a generally cylindrical or otherwise shaped member that the user of the delivery device 10 may grip during use. The handle 100 may be at least partially hollow and house various components therein, and may have one or more internal lumens so that medical devices may be passed through the delivery device 10 from the proximal end to and beyond the terminal distal end of the delivery device 10. The handle 100 may be rotatably coupled to the deflection knob 500, with the deflection knob 500 being rotatable about the central longitudinal axis of the handle 100. The deflection knob 500 may be operably coupled to two pull wires that traverse the deflectable sheath 600 and which are fixed to anchors (or pull rings or similar structures) near the distal tip of the sheath 600. Rotation of the deflection knob 500 in a first rotational direction may deflect the distal tip of the sheath 600 in a first deflection direction, while rotation of the deflection knob 500 in a second opposite rotational direction may deflect the distal tip of the sheath 600 in a second deflection direction opposite the first deflection direction. In one exemplary embodiment, the distal tip of the sheath may have a neutral angled position of about 45 degrees relative to the central longitudinal axis of the delivery device 10, with a maximum deflection (upon rotation of the deflection knob 500 in the first, e.g. clockwise, rotational direction) of about 120 degrees (shown in phantom lines in Fig. 1A) relative to the central longitudinal axis of the delivery device 10, and a minimum deflection (upon rotation of the deflection knob 500 in the second, e.g. counterclockwise, rotational direction) of about 0 degrees (shown in phantom lines in Fig. 1A) relative to the central longitudinal axis of the delivery device 10. In one example, the proximal end of each pull wire may be coupled to an axially slideable component within handle 100, where rotation of the deflection knob 500 causes the two axially slideable components to slide axially in opposite directions. Suitable pull wire mechanisms are described in greater detail in U.S. Patent No. 7,691,095, the disclosure of which is hereby incorporated by reference herein. The deflection mechanisms and ranges described above are merely exemplary, and as noted above, steering or deflection control may in some embodiments be entirely omitted from delivery device 10.

The catheter or deflectable sheath 600 may define a lumen therethrough configured to allow other devices to pass through the lumen. The catheter 600 may be formed from any suitable materials and in any suitable configuration. In one example, the catheter 600 includes an innermost liner layer, a torque transfer layer surrounding at least portions of the inner layer, and an outer sheath formed over the torque transfer layer. The wall of the catheter 600 may define lumens as well, for example two lumens spaced about 180 degrees apart, to accommodate the pull wires therethrough. Examples of suitable methods and materials for use in forming the catheter 600 are described in greater detail in U.S. Patent No. 7,914,515, the disclosure of which is hereby incorporated by reference herein.

### Catheter Stiffness

In some examples, catheter 600 may provide stable deployment with near 1:1 torque response. It may be desirable to avoid making the catheter 600 too stiff as doing so can eliminate feedback when contacting cardiac structures and/or reduce the ability of the physician to torque the catheter into position during use. In some examples, catheter 600 may include a torque transfer layer of braided wire, and the stiffness of catheter 600 may be reduced by reducing the braided wire size and/or the extrusion wall thickness of the outer sheath, while maintaining torque strength and kink resistance through braid optimization (e.g., number of wires, higher pick per inch (PPI), braid pattern, etc.). Additionally, reducing the stiffness may also serve to reduce the required forces to deflect one or more of the articulation points.

In some examples, the stiffness of the catheter may be reduced by modifying the braided wire and/or extrusion parameters. In some examples, a catheter may have an extrusion wall thickness of approximately 0.012-0.015 inches. In some examples, reducing the extrusion wall thickness to 0.009-0.011 inches may reduce the stiffness. In some examples, a braided wire may comprise a 16 wire, 0.003"×0.007" (" represents inches) flat wire braid with a relatively low PPI (e.g., between 30 and 35 picks per inch, or 33 picks per inch). Alternatively, a catheter may be formed with a reduced wire size (e.g., 0.002" × 0.005", or 0.002" or 0.003" round wire) to reduce the stiffness. In some examples, changing the braid pattern by increasing the PPI, and/or by moving to a 32-wire diamond pattern with smaller wire (e.g., 0.003" round) may decrease stiffness without affecting torque strength.

In some examples, the extrusion durometers for the outer sheath of a catheter may be modified to reduce the stiffness of the entire catheter, or specific areas of the catheter. For example, to increase flexibility in the proximal curve, which may improve alignment with the LAA, a durometer material below 63D Shore D or according to the Arkema resin standards (e.g., 35D, 40D, 45D, 50D, 55D or a blend thereof) (e.g., Arkema PEBAX^{®} 6333). Additionally, a more flexible or lower stiffness catheter will reduce the forces required to deflect the catheter, which reduces the pull wire strength requirement, and the torque required for the knob to deflect the catheter.

### Catheter Alignment and Deflection

In some examples, a delivery device may also include features for accommodating an inaccurate transseptal puncture location. For example, when the puncture is located too superiorly, it may be challenging to retroflex off the coumadin ridge to obtain the positioning needed for coaxial alignment. Additionally, for certain anatomies (e.g., a chicken-wing morphology), an out-of-plane distal curvature may be helpful for alignment. To address this, many physicians perform transseptal punctures with a different system (e.g., VERSACROSS^{®} wire, or BRK^{™} needles), and then exchange it for a steerable sheath/dilator. The added exchange is undesirable as it adds steps and prolongs the procedure. Thus, coaxial alignment may be improved via a more flexible shaft as described above by varying the braid, wire size, pick per inch, braid pattern, extrusion thickness and/or durometer, etc.

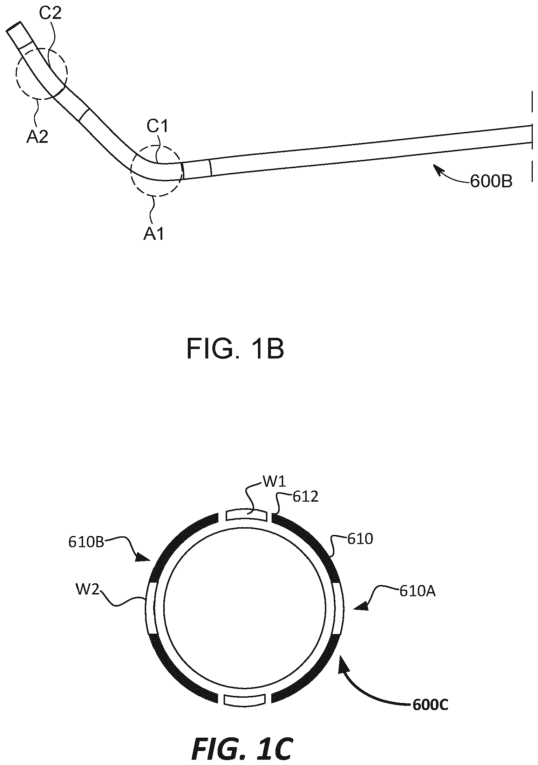

Additionally, or alternatively, a catheter 600B may be formed to have two main points of articulation, A1,A2 including a deflectable proximal curve C1 at point of articulation A1 and a deflectable distal curve C2 at point of articulation A2 (See, Fig. 1B). The first point of articulation A1 may be spaced 55-70 mm from the distal tip of catheter 600B, and the second point of articulation A2 may be spaced 10-25mm from the distal tip of catheter 600B, measured from the distal tip to the center of the curve. In some examples, the proximal and/or distal curve plane(s) of deflection may be optimized to accommodate more anatomies. In some examples, the deflectable proximal curve C1 may deflect within the same plane that it is formed in, or it can deflect out of plane, depending on the location of the pull wires. Proximal curve C1 may correspond to one or more additional knobs or steering mechanisms, and may allow the tip of the sheath to deflect in two separate planes, or four completely different directions, depending on where the pull wires are positioned along the circumference of the catheter. In some examples, proximal and distal curves C1,C2 each have two directions of deflection, which results in a four-way deflectable catheter 600B. In some examples, distal curve C2 has two directions of deflection, and proximal curve C1 has four directions of deflection, which results in a six-way deflectable catheter 600B. In some examples, distal curve C2 has four directions of deflection, and proximal curve C1 has two directions of deflection, which results in a six-way deflectable catheter 600B.

The planes of deflection of proximal and distal curves C1,C2 may also be optimized to accommodate more anatomies by changing the position of the pull wires relative to the curve plane that is formed into the sheath of the catheter. In some examples, one or more pull wire locations are offset to optimize the deflection of the catheter out-of-plane for the most challenging anatomies (e.g., chicken-wing and reverse chicken-wing). In some examples, a four-way deflectable catheter may be formed with a proximal pull ring that is larger than the lumen, liner and pull wires and lumens that run to the distal pull ring. However, this stack-up may result in a thicker wall catheter, or a bump in this area if the extrusions are not modified to accommodate the larger outer diameter at the proximal pull ring. Alternatively, one potential modification is to form a four-way deflectable catheter 600C with a split pull ring 610, having defined channels 612 between the two split halves 610A,610B defining the space where the distal pull wires W1 travel to avoid increasing the outer diameter, the pull wires W1 being orthogonal to proximal pull wires W2 (*See,* Fig. 1C). It will be understood that other non-orthogonal variations are possible. For example, the proximal pull wires may be disposed anywhere on the split pull ring (i.e., the pull wires do not have to be centered on the split pull ring), such that the proximal and distal pull wires, running in the channels created by the split ring, may be disposed close to or adjacent one another. In some examples, the split rings may be relatively large to maximize the surface area contact for reflow, and ultimately ensure the strength of the pull ring within the liner and outer polymer. The pull ring(s) may have holes in them so that a polymer can flow through the pull ring, locking it into place.

To better illustrate the deflection capabilities of a catheter 600B, a four-way deflectable catheter 600B is shown in various positions in Figs. 1D-F. It will be understood that the deflection location(s) may be adjusted by varying the location of pull rings, the radius of neutral curvature, and durometers at certain locations. In this example, catheter 600B is shown deflecting to a neutral proximal curvature (Fig. 1D) that forms an angle α1 of approximately 45 degrees with respect to the longitudinal axis of the catheter 600B. As shown in Fig. 1E, catheter 600B may also be capable of superior deflection from the neutral position, the superior deflection forming an angle α2 of approximately 0-45 degrees with respect to the longitudinal axis of catheter 600B. Also, as shown in Fig. 1F, catheter 600B may also be capable of inferior deflection from the neutral position, the inferior deflection resulting in an angle α3 of approximately 45-120 degrees or between 45-90 degrees with respect to the longitudinal axis of catheter 600B.

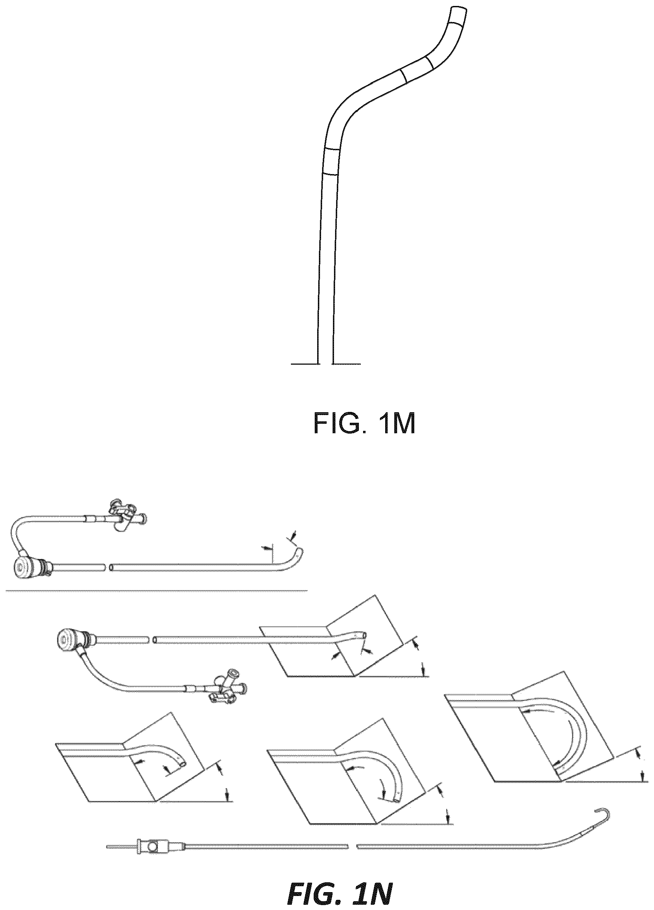

As shown in Fig. 1G, arrows G1 indicate potential direction(s) for out-of-plane deflection of the proximal curve C1 of catheter 600B. As previously noted, the out-of-plane deflection may be possible at both the proximal curve C1 and/or the distal curve C2. In Fig. 1H, an example of minimal out-of-plane superior deflection of the tip is shown. Conversely, in Fig. 1I, a more out-of-plane superior deflection of the tip is shown. Figs. 1J-M illustrate additional shapes that can be achieved with catheter 600B, such as a shape having inferior proximal deflection to deflect off the coumadin ridge and superior distal deflection to deflect into a reverse chicken-wing (Fig. 1M).

### Transseptal Punctures

By adding one or more configurations of the articulation described above (e.g., four-way catheter steering or six-way catheter steering), a catheter may provide the ability to achieve a desirable curve shape to more easily perform a transseptal puncture through the steerable catheter. Due to the required length of the proximal section once the catheter crosses the septum to reach the left atrial appendage, it may be desirable to modify the deflection initially, or start with a different neutral position before deflecting to the desired position for left atrial appendage access. In some examples, it may be desirable to maintain a neutral position as-is or in an optimal position for left atrial appendage alignment, and then deflect prior to the transseptal puncture step as needed. Alternatively, it may be desirable to achieve a neutral position optimal for a transseptal puncture. This may include an approach similar to that of a Swartz^{™} braided transseptal guiding introducer. Examples of different Swartz^{™} braided transseptal guiding introducer configurations are shown in Fig. 1N. The catheter approach for a transseptal puncture may initially be similar to that of a SL0^{™} curve, deflect further for transseptal puncture if desired, similar to SL1^{™} and SL2 ^{™} curves, and beyond. The catheter may then deflect again for left atrial appendage alignment. In this configuration, one option is to have the neutral curve indicators initially set for left atrial appendage alignment, and for the physician to rotate knobs to the neutral position after a transseptal approach. In some examples, a shortened handle and catheter working length may be desired to accommodate a conventional needle (e.g., a 98 cm BRK^{™} needle). The handle may be shortened by optimizing and/or reducing the amount of travel required for certain components (e.g., sliding blocks), eliminating dead space in the proximal end of the handle (~.25"), shortening the knobs, shortening the amount of mounting shaft between the knob and the sliding block, replacing the knob mechanism for another (e.g., toggle or slider), shortening the sliding blocks, placing one or more of the sliding blocks at different radial distances or more radially outward, shortening the modified wire guide (e.g., strain relief), and/or shortening the wire clips that secure the pull wires proximal to the sliding blocks. Additionally, or alternatively, removing the bypass valve, and the external valve housing from the catheter and internalizing it in the handle can reduce some length on the proximal end.

In some examples, instead of articulating the catheter to achieve the optimal starting position for a transseptal puncture, it may be possible to provide a dilator that is sufficiently stiff to force the sheath (at the proximal curve C1, for example) to straighten or partially straighten, and have a soft dilator (and sheath) section just distal to proximal curve C1 that allows flexion in a more typical transseptal curve shape. This may eliminate the need for the physician to articulate the sheath to straighten it out for an initial transseptal puncture shape, only to have to articulate it again for a final transseptal puncture shape. Another dilator option is to decrease the catheter outer diameter in the section of the sheath (and possibly the entire proximal shaft) that requires flexibility during transseptal puncture.

Flushing tube 400 may be a tube with a valve (e.g. luer lock) or connector at a first end thereof, with the second end of the flushing tube 400 (i.e., the end closest to handle 100) being in fluid communication with hemostasis valve assembly 200. The flushing tube 400 may be utilized to introduce fluid into and through the delivery device 10, for example to purge air out of the delivery device 10 prior to use. Flushing tubes are generally well known as they pertain to delivery devices, and thus flushing tube 400 is not described in greater detail herein.

### Dilators

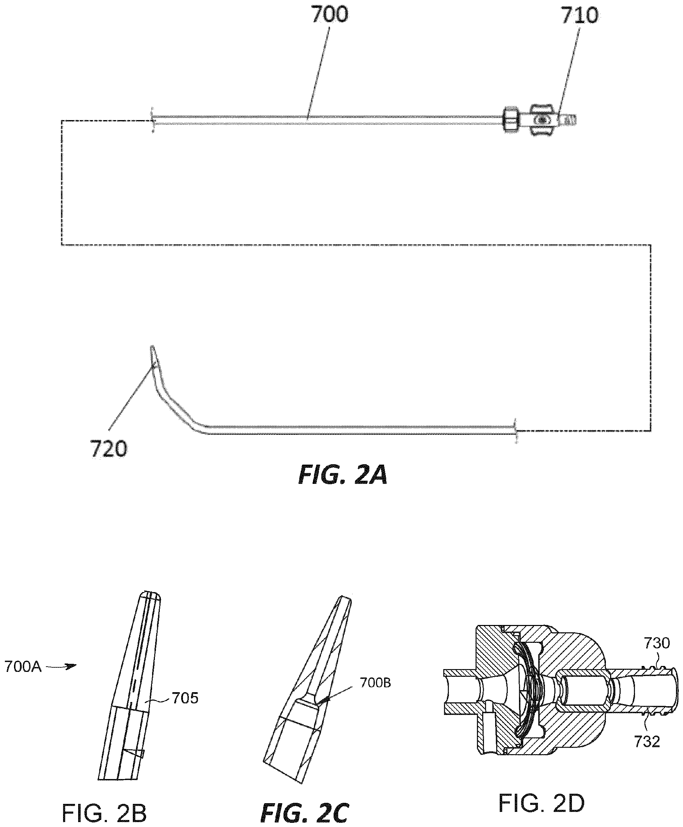

Fig. 2A illustrates a dilator 700 that may be used with delivery device 10. In the illustrated example, dilator 700 is a solid member that includes a connector 710 such as a luer lock at a proximal end thereof, and an atraumatic tip 720 at a distal end thereof. Although referred to as a "solid member," it should be understood that dilator 700 may include a guidewire lumen passing therethrough to allow for the dilator 700 to ride over a guidewire. In other words, dilator 700 may also be thought of as a "substantially solid" or thick-walled device. The distal end portion of the dilator 700 may, in the absence of applied forces, have a straight orientation, or it may form an angle of about 45 degrees relative to the central longitudinal axis of the dilator 700. In other words, the dilator 700 may include a distal portion that has a neutral angle that is about the same as the neutral angle of the distal tip of the catheter 600, whether that angle is about 45 degrees or another value. The dilator 700 may have an outer diameter that is about equal to (or slightly smaller than) the inner diameter of the catheter 600. As will be described in greater detail below, the dilator 700 may be positioned within and through the catheter 600 during delivery of the delivery device 10 to the desired anatomical location. Then the dilator 700 may be removed to allow for other devices, such as an LAA occluder, to be passed into and through the delivery device 10 for implantation.

In some examples, a modified dilator may be formed to accommodate needles and RF wires. For example, conventional dilator 700A may include a step 705 formed in the inner diameter that does not allow wires to pass through (Fig. 2B). Eliminating this step and replacing it with a taper, as shown in dilator 700B (Fig. 2C), may be desirable for wire advancement through the inner diameter. Additionally, the internal geometry of the dilator may be modified for a smoother transition to avoid skiving the inner diameter of the dilator with a transseptal puncture needle. Dilator 700B may also comprise softer materials, such as MDPE, LDPE or a softer PEBAX^{®} or Nylon, to reduce potential trauma when positioning the dilator for transseptal punctures. For example, articulating from SL0^{™} type curvatures to SL2^{™} type curvatures and scraping the dilator across the septum may be traumatic. To address this, dilator 700B maybe softer overall with a more flexible atraumatic tip. Dilator 700B may include a more robust or hard inner liner with a flexible outer layer to avoid skiving while increasing flexibility. Dilator 700B may include a stiffer proximal section for column strength for advancement through the hemostasis valve in the handle, but a softer tip to avoid trauma to the anatomy. Dilator 700B may also include a robust proximal main shaft with a more flexible section only through the sheath steerable sections to not affect steerability while the dilator is being used. A dilator adapter may also be used to center the dilator as it is inserted through the hemostasis valve to protect the valve from tearing. In one example, shown in Fig. 2D, an integrated dilator centering feature 730 having a gradually decreasing diameter or funnel 732 may be optionally added to the proximal end of the handle to help guide the dilator down the center of the valve.

### Hemostasis Valves

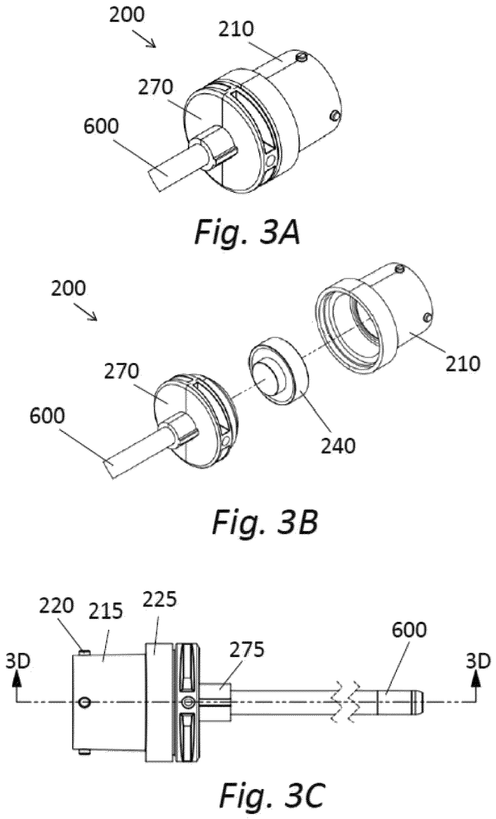

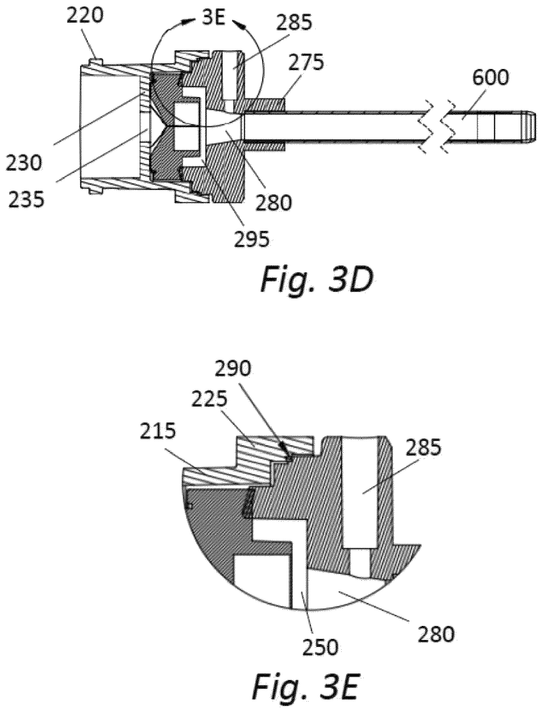

Fig. 3A shows hemostasis valve assembly 200. In the assembled condition, a cap 210 of the hemostasis valve assembly is coupled to a hub 270 of the hemostasis valve assembly, and a proximal end of the catheter 600 is coupled to a distal end of the hub 270. Although not shown in Fig. 3A, the handle 100 may be coupled to and extend from a distal end of the hub 270. Fig. 3B shows hemostasis valve assembly 200 in an exploded view, showing that the hemostasis valve 240 provides a seal between the cap 210 and the hub 270 in the assembled condition of the hemostasis valve assembly 200.

Referring to Figs. 3C-D, the proximal end of the catheter 600 may be received within and coupled to an extension 275 (*e.g.,* a cylindrical extension) extending distally from a center of the hub 270. The interior of the cylindrical extension may be open such that, in the assembled condition, the inner lumen of catheter 600 is accessible from a proximal end of hub 270, via hemostasis valve 240, as described in greater detail below. The hub 270 may form a central aperture 280, which in the illustrated embodiment, is tapered from a relatively large proximal diameter, to a relatively small distal diameter where the central aperture 280 opens to the interior of the extension 275 and to the inner lumen of the catheter 600. The hub 270 may also define a flush-port 285 that has a first end open to the exterior of the hub 270, and a second opposite end that opens to the central aperture 280. The flush-port 285 is configured to couple to flushing tube 400, so that fluid pushed through the flushing tube 400 enters the hub 270 distal to the hemostasis valve 240, allowing for flushing and/or de-airing of the interior of the delivery device 10. Referring to Figs. 3D-E, the hub 270 may also include reduced outer diameter portions 290, which may be provided as stepped down diameters that form shoulders, extending proximally. With this configuration, correspondingly sized and/or shaped distal portions of the cap 210 may be coupled to the hub 270 at those locations, for example via ultrasonic welding, with the resulting assembly having a generally smooth outer diameter between the transition from the cap 210 to the hub 270. Lastly, the hub 270 may define a generally cylindrical recess 295 at a proximal end thereof, for example radially inwardly of the stepped portions 290 and proximal to the central aperture 280. This recess 295 may be sized and shaped to receive a distal portion of the hemostasis valve 240 therein in the assembled condition of the hemostasis valve assembly 200.

Referring now to Figs. 3A-D, cap 210 may include a main body 215 at its proximal end, which may be generally cylindrical and hollow. The main body 215 may include one or more protrusions 220 extending radially outward therefrom for interaction with the hemostasis valve knob 300, described in greater detail below. In the illustrated embodiment, each protrusion 220 is a cylindrical boss, and a total of four bosses are provided at about 90 degree spacing around the outer circumference of the main body 215. However, in other embodiments, more or fewer protrusions 220 may be provided, at the same or different relative spacing, and with shapes that are similar to or different than cylindrical bosses. At its distal end, the cap 210 may transition from main body 215 to a rim 225 having a diameter that is larger than the main body 215. The interior diameter of the cap 210 at the rim 225 may also be larger than the interior diameter at the main body 215. As shown in Figs. 3D-E, the interior surface of the rim 225 may include stepped portions that form shoulders that have a shape and configuration generally complementary to the stepped portions 290 of hub 270. As described above, these complementary features may assist in fixing the hub 270 to the cap 210, for example via ultrasonic welding, although other modalities (e.g. adhesives) may be suitable for the fixation.

Referring to Fig. 3D, the cap 210 may include an interior flange 230 extending radially inwardly from the main body 215, about halfway along the length of the main body 215. The interior flange 230 may define a substantially circular aperture 235 at or near a radial center of the cap 210, such that the aperture 235 is substantially coaxial with aperture 280 and catheter 600. With this configuration, a generally cylindrical recess may be formed, the recess having an open proximal end, and being bounded by the main body 215 and, at its distal end, the interior flange 230.

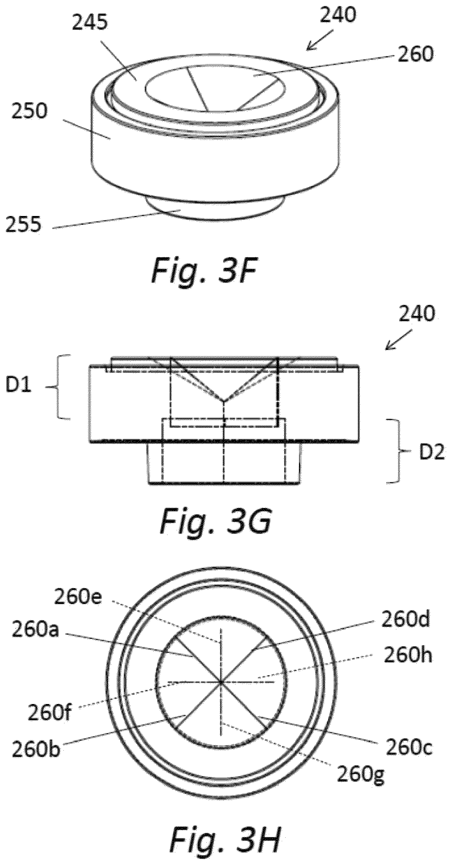

Referring now to Figs. 3B, 3D, and 3F, the hemostasis valve assembly 200 may include a hemostasis valve 240 positioned therein. As best shown in Fig. 3F, the hemostasis valve 240 may include a proximal section 245, a flanged section 250, and a distal section 255. The proximal section 245 may include a generally conical recess extending in a direction toward the distal section 255, the contours of which may assist in guiding a device into and through the hemostasis valve 240. Each of these three valve sections may have a generally circular or cylindrical shape (which may or may not include a taper), with the flanged section 250 having a larger outer diameter than the proximal section 245 and the distal section 255, and the distal section 255 having a smaller outer diameter than the proximal section 245.

The hemostasis valve 240 is preferably formed as a single integral member, and one or more cuts or slits are formed therein to create the actual valve functionality. One particular way of creating the valve functionality is described directly below, but it should be understood that other methodologies and other resulting valve structures may be suitable for use instead of the particular example shown and described herein. For example, Figs. 3G-H are side and top views, respectively, of the hemostasis valve 240 with slits made therein illustrated. In this particular example, four slits are formed in the proximal section 245 extending toward the distal section, and four slits are formed in the distal section 255 extending toward the proximal section, each group of four slits being formed in an "X" configuration at a spacing of about 90 degrees between adjacent slits, with the two groups of slits being offset rotationally from each other by about 45 degrees.

In particular, referring to Figs. 3G-H, four slits 260a-d are formed in the proximal section 240, each slit 260a-d extending a depth D1 toward the distal section 255. Each slit 260a-d is spaced about 90 degrees from an adjacent slit to form the cross or "X"-shape shown. These slits 260a-d may be thought of as forming flaps 260, labeled in Fig. 3F, having generally triangular or wedge shapes. The depth D1 may extend a depth into the flange section 250, but stop short of the distal section 255. The four slits 260a-d intersect at a central intersection point that extends a distance or depth to form a line where the slits intersect. A second group of slits 260e-h are formed in the distal section 255 extending a depth D2 toward the proximal section 245, having substantially the same configuration as slits 260a-d, except being offset, for example by about 45 degrees, relative to slits 260a-d. In particular, as shown in Fig. 3H, the four slits 260e-h form a cross or "X"-shape, with each slit spaced about 90 degrees from an adjacent slit in the group, and the four slits 260e-h meeting at a central intersection point that extends a distance of depth to form a line where the slits intersect. The pathway between the proximal section 245 and the distal section 255 is completed by the two intersection lines of the two groups of slits 260a-d, 260e-h both overlapping for the small distance by which depths D1 and D2 overlap, as shown in Fig. 3G.

When the hemostasis valve assembly 200 is assembled, the outer circumference of the flanged section 250 may be in contact with an inner surface of the main body 215 of the cap 210, just distal to the interior flange 230. The proximal section 245 of the hemostasis valve 240 may be in contact with a distal surface of the interior flange 230, with the center of the hemostasis valve 240, where the flaps 260 converge, substantially coaxial with the circular aperture 235 defined by the interior flange 230, as best shown in Fig. 3D. The distal section 255 of the hemostasis valve 240 may extend into the cylindrical recess 295 of the hub 270. As best shown in Fig. 3D, the distal section 255 may include an outer rim that is substantially coaxial with the central aperture 280 of the hub 270.

When the hemostasis valve assembly 200 is assembled, the connection between cap 210 and hub 270 is fluid-tight such that, in order for any fluid (or other objects) to pass into the cap 210 and through the hub 270 to the catheter 600, the fluid must pass through hemostasis valve 240. In the absence of applied forces, the flaps 260 of the hemostasis valve 240 create a fluid-tight seal so that fluid is prevented from passing through the hemostasis valve 240. It should be understood that hemostasis valves that have other specific configurations than that shown may be suitable for use with the hemostasis valve assembly 200.

Typically, hemostasis valves such as hemostasis valve 240 have a soft durometer, for example from about 20 - 70 Shore A durometer, and are typically formed of silicone and/or urethane and/or other similar materials. If a hemostasis valve is intended to allow a relatively large device to pass therethrough, the hemostasis valve will typically require a relatively large diameter and/or a relatively large thickness. As hemostasis valves get larger and/or thicker, it may require more force to push a device through the seal, for example because the seal may provide greater resistance against such passage. Also, at least partially because of the low or soft durometer of the material forming a hemostasis valve, one or more drops of silicone oil (or other lubricant) are typically provided by the valve manufacture in the slits to help ensure that the flaps do not stick together, particularly if the valve is sitting on a shelf for a period of time between manufacture and use. The lubricant may be applied directly to the material of the valve or in other embodiments the lubricant may infuse or self-leach into the material. The requirement for a device to have a relatively large column force to easily pass through a hemostasis valve, as well as the possible contamination of that device with pre-applied or infused silicone oil (or another lubricant) as it passes through the valve, may be generally undesirable features, depending on the particular device being passed through the seal. The hemostasis valve assembly 200 described above, in combination with the hemostasis valve knob 300 described below, may overcome one or both of these possible undesirable features.

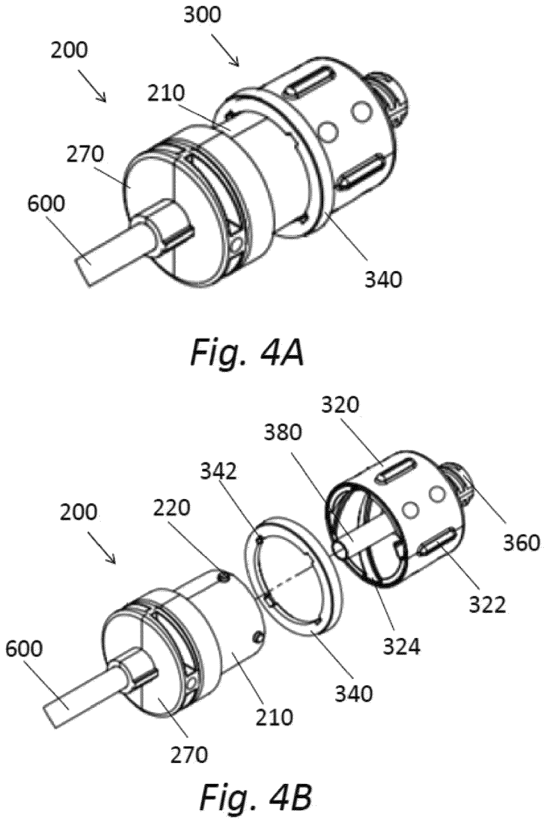

Fig. 4A shows the hemostasis valve assembly 200 assembled to the hemostasis valve knob 300, with Fig. 4B showing a corresponding exploded view. The hemostasis valve knob 300 may include a main body 320, a retaining ring 340, a bypass hub 360, and a bypass tube 380.

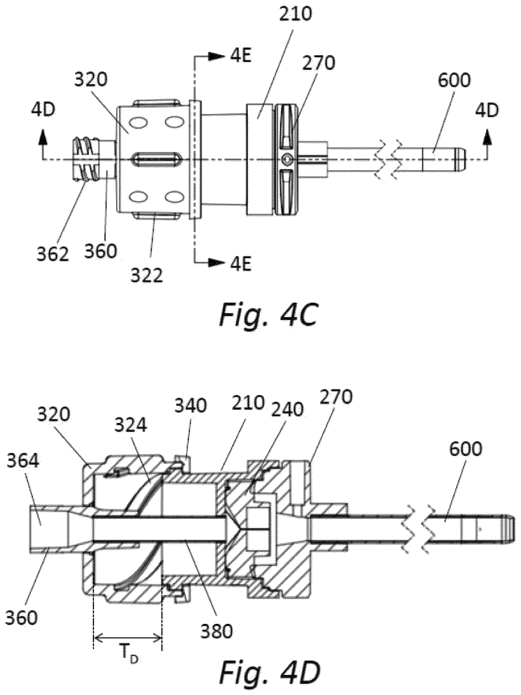

Referring generally to Figs. 4A-D, the main body 320 may be generally cylindrical and may include texturization on an outer surface to enhance a user's grip on the main body 320. In the illustrated example, the main body 320 includes four raised knurls 322 at equal circumferential spacing to assist a user in torqueing the main body 320. However, it should be understood that other numbers, types, and spacing of texturization features may be provided instead of the raised knurls 322. The main body 320 may have a substantially open distal end, and an inner diameter that is sized to fit over the outer diameter of the main body 215 of cap 210. As best shown in Figs. 4B and 4D, the inner surface of main body 320 may include a plurality of curved channels or recesses 324, for example each in a generally helical configuration. Each curved recess 324 may extend to the terminal distal end of the main body 320, and may have a width and a depth sized to receive a corresponding protrusion 220 therein. With this configuration, when the main body 320 is assembled over the main body 215, and each protrusion 220 is received within a corresponding curved recess 324, rotating the main body 320 will translate the hemostasis valve knob 300 toward or away from the hub 270 of the hemostasis valve assembly 200, as described in greater detail below. For example, as shown in Fig. 4D, rotation of the main body 320 allows for distal translation or advancement of the main body 320 (along with the bypass hub 360 and bypass tube 380) a maximum available travel distance T_{D}, until the interior proximal face of the main body 320 contacts that proximal end of the cap 210. The actual available travel distance may be smaller, depending on the axial length of the recesses 324, for example. Although four curved recesses 324 are shown, more or fewer may be provided, preferably with equal number and spacing as the protrusions 220. And although referred to as a knob that is rotatable, the hemostasis valve knob 300 may also be referred to as an actuator that activates by rotation or other non-rotational movements.

Retaining ring 340 may be a generally annular member that is sized to mate with the terminal distal surface of the main body 320. In particular, the retaining ring 340 may have a distal face with an inner diameter that is slightly smaller than the outer diameter of the main body 320, and an outer side wall that has an inner diameter that is about equal to or slightly larger than the outer diameter of the main body 320. As shown in Fig. 4D, this size configuration allows the retaining ring 340 to snap over the terminal distal end of the main body 320. As best illustrated in Figs. 4A-B, the retaining ring 340 may include a plurality of recesses 342 in the distal face thereof, preferably in the same number and relative spacing as protrusions 220 and curved recesses 340. The recesses 342 are sized and spaced so that, when each recess aligns with a corresponding protrusion 220, the retaining ring 340 may slide axially over the main body 215 of cap 210. However, if the recesses 342 are not aligned with corresponding protrusions 220, there is not enough clearance for the retaining ring 340 to slide axially past the protrusions 220. This configuration may help with assembling the main body 320 to the main body 215, with the retaining ring 340 ensuring that the main body 320 cannot disconnect from the main body 215. For example, during assembly, the retaining ring 340 may be oriented with recesses 342 aligned with protrusions 220 and slid distally over the main body 215. Then, the retaining ring 340 may be rotated, for example about 45 degrees, so that the recesses 342 no longer align with the protrusions 220. Then, the main body 320 may be coupled to the main body 215 with the protrusions 220 received within curved channels 324. The main body 320 may then be fixed to the retaining ring 340, such that the terminal distal ends of the curved recesses 324 are out of alignment with the recesses 342 of the retaining ring 340. The method for fixing may be any suitable method, including adhesives, ultrasonic welding, *etc.* With this configuration, the retaining ring 340 prevents the main body 320 from slipping off the main body 215 as it moves proximally away from the main body 215 upon rotation. Fig. 4E illustrates the coupling of the retaining ring 340 to the main body 320, with other components omitted for clarity. As can be seen, the recesses 342 of the retaining ring 340 are out of alignment with the ends of the curved recesses 324 in the main body 320.

Referring to Figs. 4C-D, the hemostasis valve knob 300 includes a bypass hub 360 and a bypass tube 380 extending through a proximal surface of the main body 320. The bypass hub 360 may have a generally cylindrical outer surface, and may include threads 362 or another mechanism to facilitate coupling to other devices used in conjunction with delivery device 10. The bypass hub 360 may be formed integrally with, or formed separately and then coupled to, the main body 320. The bypass hub 360 may define a lumen 364 therethrough, and the lumen 364 may be tapered in the distal direction. The lumen 364 is preferably coaxial with the other lumens and openings within the hemostasis valve assembly 200 such as aperture 235, aperture 280, the overlapping openings of hemostasis valve 240, and is also preferably coaxial with the catheter 600.

Referring now to Fig. 4D, the bypass tube 380 extends distally from the bypass hub 360. The bypass tube 380 is preferably generally cylindrical with an outer diameter that is about equal to or just smaller than the interior diameter of aperture 235. The bypass tube 380 may be formed integrally with the bypass hub 360, for example via injection molding, in which case suitable materials may include acrylonitrile butadiene styrene ("ABS"). In other embodiments, the bypass tube 380 may be formed of materials such as polyoxymethylene (e.g. under the tradename Delrin^{™}), etched polytetrafluoroethylene, polyether block amide (e.g. under the tradename Pebax^{™}), or other suitable materials such as Nylon (e.g. lined Nylon 12 tubing). The bypass tube 380 preferably has a relatively high column strength, such that it may easily pass through hemostasis valve 240 without buckling or otherwise being damaged as it translates distally. For example, the bypass tube 380 may have a column strength that is greater than the column strength of the medical device that is to be passed through the bypass tube. The bypass tube 380 preferably has a length so that, when the main body 320 is in its proximalmost position relative to the cap 210, the distalmost end of the bypass tube 380 is positioned within aperture 235 just proximal of the hemostasis valve 240.

It should be understood that the hemostasis valve assembly 200 and knob 300 are illustrated in Figs. 4A, 4C, and 4D in a sealed or first condition, in which the main body 320 is in its proximalmost position relative to the cap 210. In this condition, as noted above, the distalmost end of the bypass tube 380 is positioned just proximal to the hemostasis valve 240, so that the bypass tube 380 does not traverse the hemostasis valve 240. A user may transition the hemostasis valve assembly 200 and knob 300 into an open or second condition by rotating the main body 320 relative to the cap 210, forcing the hemostasis valve knob 300 to translate distally until the bypass tube 380 passes through the hemostasis valve 240. This open or second condition is illustrated in Fig. 4F. As shown in Fig. 4F, the main body 320 of the hemostasis valve knob 300 has been rotated to advance the main body 320, as well as the retaining ring 340, bypass hub 360, and bypass tube 380 distally relative to the hemostasis valve assembly 200. The completion of the advancement can be seen by, for example, comparing the available travel distance T_{D} shown in Fig. 4D having been decreased to substantially zero, with the inner face of the proximal main body 320 abutting the proximal end of cap 210. In this second or open condition, the bypass tube 380 fully traverses the hemostasis valve 240, with the distal terminal end of the bypass tube 380 positioned within or adjacent to central aperture 280. Thus, in the first or sealed condition shown in Fig. 4D, the hemostasis valve 240 is closed and the lumens or openings distal to the hemostasis valve 240 are sealed from the lumens or openings proximal to the hemostasis valve 240. However, in the second or open condition shown in Fig.4F, the bypass tube 380 forces the hemostasis valve 240 to remain open, putting the lumens or openings distal of the hemostasis valve 240 in fluid communication with the lumens or openings proximal to the hemostasis valve 240.

### Alternative Hemostasis Valve Configurations

As described above, a hemostasis valve may be used to minimize blood loss, prevent coronary air embolism, and/or facilitate the delivery system into the body. In some examples, a saline drip may be used to assist with hemostasis valve bleed. A saline drip may be undesirable, as it can increase the volume of fluids in the patient, which can affect the heart size and ultimately the sizing of a device. Additionally, saline drips and extra connections may increase the chance of air ingress, and the complexity and time it takes to complete a procedure.

In some examples, an alternative valve configuration or Tuohy-Borst valve on the proximal end of the system may eliminate the need for saline drip, and allow for aspiration through the sheath to eliminate any air that does enter. Additionally, a check valve and/or flow control switch or stopcock may allow for dilator (or other instrument) introduction without introducing air into the sheath. A 2-way stopcock or other valve may be used to replace a flow control switch. In these examples, the alternative valve may allow for the introduction of a plurality of instruments ranging from a 0.035" guidewire to a 17F outer diameter tubing while maintaining a complete seal. Additionally, certain configurations may allow for the removal of the bypass hub and/or bypass tube. Removal of the bypass feature may allow for a single operator to manipulate the system and pass any one or more of guidewires, dilators, delivery sheaths, and devices in a loader tube through the hemostasis valve.

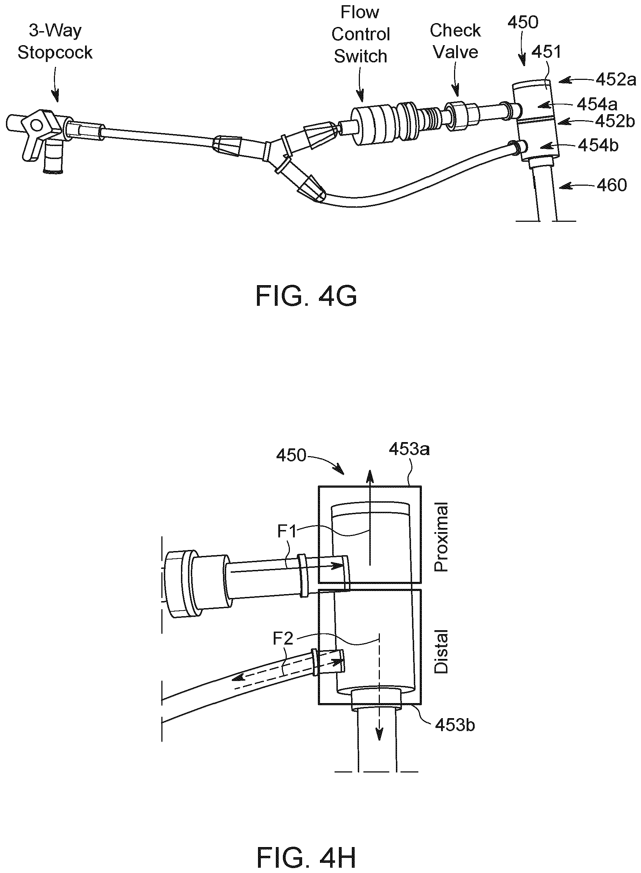

Fig. 4G-H illustrate a first embodiment of a valve assembly 450 having a two-seal configuration that allows for a lumen to be flushed and aspirated while maintaining the ability to achieve a wet-to-wet connection. This configuration may improve physician ease-of-use and eliminate concern for air ingress during a left atrial appendage occlusion procedure. Two seals are provided, and either may independently seal down to a 0.035" guidewire and up to a 17F outer diameter loader tube. This sealing capability, combined with using clear or transparent material for valve housing proximal to the shaft handle and allowing aspiration, may reduce the occurrence of air embolisms.

Specifically, valve assembly 450 may include two independent seals; a proximal seal 452a, and a distal seal 452b and utilize two different flush-ports 454a,454b. The first flush-port 454a may be disposed between the two seals 452a,452b and the second flush-port 454b being disposed distal to the second seal 452b (e.g., closer to lumen 460 of the catheter). In this example, valve assembly 450 includes a valve body 451 that is transparent to allow for improved visibility. Proximal seal 452a may be configured to seal an instrument ranging from a 0.035" guidewire to a 17F outer diameter loader tubing. First flush-port 454a may be in line with a check valve and/or a flow control switch to regulate the fluid flow to the proximal body 453a of the valve as shown by arrows F1 in Fig. 4H. When the flow control switch is open and the end of the lumen tubing is blocked, fluid can flow out of the valve, following arrows F1, thus allowing for a wet-to-wet connection during a procedure (e.g., when inserting the dilator). Distal seal 452b may also seal a plurality of instruments ranging from a 0.035" guidewire to a 17F outer diameter loader tubing. Second flush-port 454b may be distal to the distal seal 452b and may allow for flushing of distal body 453b through the lumen as shown by dashed arrows F2. In some examples, both flush-ports 454a,454b are connected to a 3-way stopcock for ease of use. Because the check valve does not allow aspiration, second flush-port 454b may be an outlet for aspiration. With second flush-port 454b allowing aspiration and the presence of distal seal 452b, the distal body 453b and lumen body may be made free of air without needing to use the proximal body.

Fig. 4I-1 to Fig. 4I-3 illustrates a second embodiment of a valve assembly 470. In this example, a Tuohy-Borst valve 470 may be used to create a complete seal when no instrument is present or around an instrument. In some examples, valve 470 can seal a plurality of instruments ranging from a 0.035" guidewire and to a 17F outer diameter loader tube. This configuration may give physicians the ability to alter the size of the seal depending on the need during a procedure. Specifically, a Tuohy-Borst hemostatic valve may allow for the introduction of multiple-sized components of a delivery system while maintaining a tight seal. The mechanism of opening and closing the seal allows the physician to control the amount of back bleed during the procedure. For example, the valve may gradually close by rotating the outside knob clockwise and may seal completely in one full turn or less. Figs. 4I1-4I3 illustrate the maximum and minimum capabilities of the Tuohy-Borst hemostatic valve including a complete seal with nothing in the valve (Fig. 4I-1), a seal around a 0.035" guidewire (Fig. 4I-2) and a seal around a 17F outer diameter tube (Fig. 4I-3). As previously described, a transparent Tuohy-Borst valve may be integrated at a proximal end of a handle, distal to the valve, for visibility into the valve housing.

Fig. 4J illustrates an hourglass valve assembly 480 configured to allow for the lumen body to be flushed through the valve housing separate from the seals, while also allowing aspiration. Specifically, the hourglass shape of the valve assembly 480 incorporates two sealing elements including a proximal seal 482a and a distal seal 482b. Proximal and distal seals 482a,482b may both be capable of sealing around a plurality of instruments ranging from a 0.035" guidewire to a 17F outer diameter loader tube (e.g., guidewires, dilators, delivery sheaths and loaders). Notably, in some examples, distal seal 482b may be capable of providing a complete seal without an instrument present, while proximal seal 482a does not. The hourglass valve 480 may be partially or fully enclosed in clear housing 481 to allow for increased visibility. In some examples, a port 484 positioned between the two seals (i.e., distal to proximal seal 482a and proximal to distal seal 482b) may allow flushing through the rest of the valve housing into the lumen body as shown by arrow F3.

Such valve assemblies may be useful in shortening a handle, and one example of a shortened handle 100B is shown in Fig. 4K-1. A handle 100B may include a valve assembly 490 according to any of the configurations described above, incorporated into the handle 100B, and an ultrasonically welded cap 492 securing the valve assembly 490. An overmolded transparent hub 494 may couple to the handle body 495, and a flush-port 496 may extend through the transparent hub 494. Handle body 495 may also include a longitudinal handle cutout or slit 497, and a transparent tubing 498 disposed therein and connecting to a catheter or deflectable sheath 499. In some examples, the slit 497 may be between .25 inches and 2 inches in length. By using transparent materials for certain components (e.g., the valve assembly, hub, tubing, etc.), a physician may be confident that no air resides in the system through direct visualization. Fig. 4K-2 is a schematic representation of one example of a handle showing a slit 497 distal to the valve assembly 490 and transparent tubing 498.

### Handle Configurations

In Fig. 1A, deflection knob 500 is shown at a distal end of handle 100 adjacent outer sheath or catheter 600. In some examples, one or more knobs may be disposed more proximally on the handle to allow for physicians to have their hands closer together when moving from deflection to device deployment. As shown in Fig. 4L, handle 100C may include a proximal knob 500A and a distal knob 500B, and a neutral curve indicator 502 disposed on an immovable portion of the handle in the form of a raised ridge,bump, recess, pad-printed element or laser marked element. Neutral curve indicator 502 may be positioned on the handle 100C to indicate a neutral position of the catheter 600 for transseptal puncture and/or LAA alignment. In this example, each of proximal and distal knobs 500A,500B may include a designated neutral position indicator 504a,504b, such that aligning each indicator 504a,504b with neutral curve indicator 502 will signal to the operator that the corresponding curve on the catheter is in the neutral position. In use, the physician may utilize tactile feedback and appreciate the positions of one or more of the knobs (and thus the proximal and distal curves) with respect to the neutral position without looking at the handle. Other indicators may be added to the handle to indicate optimal curve shapes for a particular procedure or procedure step. It will be understood that a handle may include one, two, three or more knobs. For example, a six-way adjustable deflectable sheath may include two designated knobs to actuate one of the curves (e.g., the proximal or distal curve) in four directions.

Figs. 4M-N illustrate two examples of loader assemblies. In Fig. 4M, a loader assembly 510a includes a loader coupler 515a attached to a loader tubing 516a. The loader tubing 516a may be at least partially moved within the handle and at least partially disposed therein, with at least a portion (e.g., greater than 0.25 inch) extending distally through the valve, and loader coupler 515a may be connected to the handle after insertion of the loader tubing, resulting in a shorter overall system length. In another example, shown in Fig. 4N, a second loader assembly 510b includes a coupler 515b configured to mate with the handle, and a longer loader tube 516b may extend through the valve and between 3 inches and 5 inches (e.g., 4.75 inches) into the sheath 520, the sheath having a larger outer diameter to accommodate the loader tubing. The sheath 520 may include a taper 522 to reduce the inner diameter of the sheath from a first diameter to a second diameter. In at least some examples, at least 30%, 50% or 66% of the loader tubing 516b may be disposed within the handle.

### Additional Modifications to the Delivery Device

Other variations are possible such as reducing the number of connections between components of the delivery device. For example, a conventional device may include connecting a 12F-14F adapter to a sheath, the loader to the adapter, a 2x-1x adapter to the loader, a valve (e.g., a Tuohy-Borst valve to the adapter), and a stopcock to the Tuohy-Borst valve. Multiple connections increase the risk of leak and air ingress. Removing as many of these connections as possible will improve air management and confidence that the system is hermetically sealed. In some examples, a delivery system may include any or more of the following modifications: (i) providing only a 14F sheath to eliminate the 12F-14F adapter, (ii) eliminating the 2x-1x adapter by varying the occluder design and molding the smaller luer profile directly on to the hub, (iii) removing the luer connection on the hub, and replacing it with the Tuohy-Borst rotating collar, so that the Tuohy-Borst collar can be directly snapped into the loader hub, and/or (iv) removing the stopcock-to-Tuohy-Borst connection and replacing it with bonded tubing that is bonded to a stopcock, a bonded stopcock directly onto the Tuohy-Borst, or an injection molded stopcock housing integrated into the Tuohy-Borst sideport.

Additional features may also be included to increase ease of use and improve ergonomics. These may include any of the following: (i) providing a different deflection mechanism (e.g., a toggle or slide) to shorten the handle, (ii) providing one or more curve deflection indicators to quickly show an indication of how much the sheath is deflecting (or the shape of the sheath) during use, (iii) rounding a loader tip for ease of insertion to reduce the force to insert it through the valve, (iv) providing thinner or softer valve material for reduced insertion forces and improved compliance, (v) modifying the flush-port location for ease of air removal/aspiration, (vi) providing a more rigid loader/Tuohy to eliminate flex, as it can be difficult to hold tension with the left hand while advancing/deploying the cable/device with the right hand if the loader/Tuohy is flexing, (vii) adding a marking on the cable to indicate to the physician when the device is nearing the end of the sheath for reduced fluoroscopy/radiation exposure, and/or (viii) removal of the luer on the proximal end and replacing it with a short quick connect.

Fig. 5A is a perspective view of an occluder 1000, which may be a LAA occluder. Very generally, occluder 1000 includes an occluding material forming a tubular structure 1030, the occluding material being a braided metal fabric, which may be formed by braiding a plurality of strands 1035, which may be strands of nickel titanium alloy (e.g. under the tradename Nitinol), together and shape set (e.g. via heat setting) to the shape shown in Fig. 5A. When in the shape-set or expanded condition (e.g. in the absence of applied forces), the occluder 1000 may include a generally disk-shaped portion 1080 configured to abut an ostium of the patient's LAA, and a second generally cylindrical portion 1085 configured to be received within the patient's LAA. In some embodiments, the disk-shaped portion 1080 may be coupled to the cylindrical portion 1085 via a small diameter transition portion. The cylindrical portion 1085 may include one or more hooks 1020 configured to engage tissue within the LAA upon deployment. The disk-shaped portion 1080 may include a connector 1040, which may for example include internal threads, for coupling to a delivery cable 1300.

Fig. 5B illustrates occluder 1000 in a collapsed condition, having a collapsed diameter D_{C} smaller than the diameter of the disk-shaped portion 1080 and the cylindrical portion 1085 when in the expanded condition shown in Fig. 5A, and a collapsed length Lc longer than the length of the occluder in the expanded condition shown in Fig. 5A. Fig. 5C illustrates the delivery cable 1300 coupled to the connector 1040, for example via a threaded hub 1350 of the delivery cable 1300 that has been threaded into the connector 1040.

An exemplary use of the delivery device 10 for delivering occluder 1000 is described below. At the beginning of the procedure (or in preparation thereof), the delivery device 10, including dilator 700, may be removed from sterile packaging. The user preferably confirms that the hemostasis valve knob 300 is in the first, sealed position such that the hemostasis valve 240 is closed. If the hemostasis valve knob needs to be adjusted, the user may rotate the hemostasis valve knob 300, for example by turning it counter-clockwise relative to the hemostasis valve assembly 200, to retract the bypass tube 380 so that it does not pass through the hemostasis valve 240. Alternatively, an alternative valve configuration such as those disclosed herein, including a passive hemostasis valve assembly may be used, which does not require adjustment. The catheter 600 and the dilator 700 may be wiped with sterile gauze dampened with sterile saline to remove any foreign material that may be on the components. The user may then pass the dilator 700 through the delivery device 10 until the distal end of the dilator 700 passes the distal end of the catheter 600. Even though the hemostasis valve knob 300 is in the first, sealed position, the dilator 700 has enough column strength to readily pass through the hemostasis valve 240 without assistance of the bypass tube 380, without any buckling or damage occurring to the dilator 700. When the dilator 700 is fully inserted through the delivery device 10, the connector 710 (or a threaded collar associated with the connector 710) may be rotated to couple to the bypass hub 360, for example by inner threads of the connector 710 engaging outer threads 362 of the bypass hub. Other loader assemblies, such as those described with reference to Figs. 4M-4N, may be used to facilitate this process, or to shorten the handle for easier manipulation. The dilator 700 is now coupled to the remaining portions of the delivery device 10 as a single unit. Access may be gained to the patient via any suitable method, and a guidewire may be advanced into the patient's vasculature until it reaches the patient's left atrium, LAA, or pulmonary vein. Prior to or during introduction of the guidewire, a puncture may be made through the patient's atrial septum if the delivery route is, for example, through the patient's femoral vein and to the right atrium via the inferior vena cava, with the septal puncture allowing the guidewire and other components to traverse the atrial septum into the left atrium. With the guidewire in place, the dilator 700 (and the remainder of the delivery device 10 to which it is coupled) may be advanced over the guidewire into the patient until the distal end of the delivery device 10 reaches the left atrium or LAA. With the distal end of the delivery device 10 in the desired location, the connector 710 may be rotated to decouple it from the bypass hub 360, and the dilator 700 may be withdrawn from the catheter 600, preferably slowly to prevent any ingress of air. After the dilator 700 is fully removed, the guidewire may next be fully removed from the delivery device 10 and the patient, although the guidewire may instead be simultaneously removed with the dilator 700.

With the distal end of the catheter 600 in the desired position, the occluder 1000 may be introduced into and through the delivery device 10. As shown in Fig. 5D, a loading tube 1400 provided with the occluder 1000 may be coupled to the bypass hub 360, for example by rotating a threaded connector 1450 (or a threaded collar associated therewith) onto the bypass hub 360. The occluder 1000 may be pushed through the loading tube 1400 toward the hemostasis valve assembly 200 while the occluder 1000 is in the collapsed condition, by pushing delivery cable 1300. Prior to the occluder 1000 reaching the hemostasis valve 240, the user rotates the hemostasis valve knob 300, for example in clockwise direction, so that the bypass tube 380 advances into and through the hemostasis valve 240. However, in some embodiments, it may be appropriate to activate the bypass mechanism to get a wet-to-wet connection with the loading tube 1400 prior to the occluder 1000 reaching the hemostasis valve 240. Either way, with the hemostasis valve knob 300 in the second, open condition, the user may push the cable 1300 to advance the collapsed occluder through the bypass tube 380, bypassing the need for the occluder 1000 to contact the hemostasis valve 240 directly as the occluder 1000 passes through the hemostasis valve assembly 200. At any point after the occluder 1000 has advanced distally beyond the hemostasis valve 240, and prior to the occluder 1000 being deployed from the catheter 600, the hemostasis valve knob 300 may again be rotated to revert the hemostasis valve knob 300 into the first, closed condition. The cable 1300 preferably is able to readily pass through the closed hemostasis valve 240 without the assistance of the bypass tube 380. The user may continue pushing the delivery cable 1300 until the occluder reaches the distal end of the catheter 600, and then deploy the occluder 1000 from the distal end of the catheter 600, allowing the occluder 1000 to self-expand into the LAA to occluder the LAA. This process may be coupled with steering of the catheter 600 using the handle 100 and deflection knob 500, if steering capabilities are included in the delivery device 10. In some examples, the user may use two or more knobs to steer the catheter 600, deflecting the catheter at one or more of the proximal and distal curves C1,C2 to achieve some of the shapes shown in Figs. 1D-M. In some examples, the user may manipulate pull-wires through a knob or other mechanism to actuate proximal and distal curves C1,C2 in two directions of deflection, which results in a four-way deflectable catheter. Six-way deflection is also possible with two directions of deflection at the proximal curve C1 and four directions of deflection at distal curve C2, or vice versa. With the occluder 1000 deployed in the desired position, the cable 1300 may be decoupled from the occluder 1000, for example via rotation of the cable 1300, and the cable 1300 may be withdrawn from the delivery device 10.

As described above, there are at least two benefits that may be provided by the hemostasis valve bypass components. First, various medical devices that are collapsible for delivery through a catheter may have low column strength, especially compared to components like dilator 700. Occluder 1000 is formed of a braided mesh of strands of nickel titanium alloy, which may result in the occluder 1000 having a low column strength. Without the bypass tube 380, as the user pushed the occluder 1000 through the hemostasis valve 240, the occluder 1000 might buckle and become damaged as it encounters resistance from the hemostasis valve 240. Further, if the hemostasis valve 240 has silicone oil or another lubricant from when it was manufactured, that silicone oil may transfer to the occluder 1000, effectively contaminating the occluder with that substance. The bypass tube 380 solves both of these problems, by eliminating resistance that the occluder 1000 would encounter from the hemostasis valve 240, as well as ensuring no transfer of contaminants occurs since there is no direct contact between the occluder 1000 and the hemostasis valve 240. Further, the embodiments disclosed herein allow for the hemostasis valve assembly 200 and hemostasis valve knob 300 to be integrated with the remainder of the delivery device 10. In other words, the functionality of the bypass tube 380 is provided without the need for another separate device beyond the delivery device 10, increasing the convenience and decreasing the procedure time that would be required by having the bypass tube 380 a fully separate component.

Although the features (e.g., the hemostasis valves, the deflecting curves, the handle configurations, etc.) described herein may be particularly useful for the LAA occluder 1000 device described herein, it should be understood that this particular use is merely exemplary. For example, any of the features described herein may be particularly useful for other occluders formed of braided metal, including septal occluders, patent ductus arteriosus ("PDA") occluder devices, patent foramen ovale ("PFO") closure devices, paravalvular leak closure devices, *etc.* It should further be understood that the features described herein may work well with other occluders, including those not formed from a braided mesh, or any other medical device, whether an occluder or not and whether formed of braided mesh or not, if that medical device has relatively low column strength and/or if it would be undesirable for that medical device to be contaminated with silicone oil or another lubricant from direct contact with a hemostasis valve. Additionally, the disclosed features may be formed with others in various combinations.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A delivery device comprising:
a handle;
a catheter sheath defining a lumen and extending distally from the handle; and
a hemostasis valve positioned at least partially within the handle, the hemostasis valve being located proximal the catheter sheath, the hemostasis valve including a first seal and a second seal, and a first flush-port disposed between the first seal and the second seal.

2. The delivery device of claim 1, wherein the hemostasis valve further includes a second flush-port disposed distal to the second seal, preferably wherein the second flush-port is in communication with the lumen of the catheter sheath.

3. The delivery device of claim 1, further comprising at least one of a check valve and a flow control switch in fluid communication with the first flush-port.

4. The delivery device of claim 2, further comprising a 3-way stopcock in fluid communication with both the first flush-port and the second flush-port, preferably wherein the first flush-port is configured and arranged to introduce a fluid to a proximal body of the hemostasis valve, and the second flush-port is configured and arranged to introduce a fluid to a distal body of the hemostasis valve, the proximal body and the distal body being separated by the second seal.

5. The delivery device of claim 1, wherein the hemostasis valve is hourglass-shaped, and / or wherein the homeostasis valve is transparent.

6. The delivery device of claim 1, wherein each of the first seal and the second seal is configured and arranged to provide an air-tight seal around one of a plurality of instruments ranging in size from a 0.035" guidewire to a 17F tube.

7. A delivery device comprising:
a handle;
a catheter sheath defining a lumen and extending distally from the handle, the catheter sheath having a deflectable proximal curve and a deflectable distal curve spaced from the deflectable proximal curve;
at least one pull ring formed within the catheter sheath;
one or more pull wires coupled to the at least one pull ring; and
a first deflection knob for actuating the catheter sheath at the deflectable proximal curve and a second deflection knob for actuating the catheter sheath at the deflectable distal curve.

8. The delivery device of claim 7, wherein the first deflection knob is coupled to the handle, the first deflection knob being rotatable about a longitudinal axis of the handle to activate the one or more pull wires.

9. The delivery device of claim 7, wherein the deflectable proximal curve is configured to deflect in two separate planes or wherein the deflectable distal curve is configured to deflect in a single plane.

10. The delivery device of claim 7, wherein the deflectable proximal curve is capable of four-way deflection, and the deflectable distal curve is capable of two-way deflection.

11. The delivery device of claim 7, wherein the deflectable proximal curve has a neutral position that forms a 45-degree angle with a longitudinal axis of the catheter sheath.

12. The delivery device of claim 8, further comprising a neutral position indicator disposed on the handle, preferably wherein the neutral position indicator comprises a raised ridge on the handle.

13. The delivery device of claim 12, further comprising a dedicated knob indicator disposed on each of the first deflection knob and the second deflection knob, each dedicated knob indicator being alignable with the neutral position indicator to signal a neutral position of one of the deflectable proximal curve and the deflectable distal curve.

14. A delivery device comprising:
a handle;
a catheter sheath defining a lumen and extending distally from the handle;
a hemostasis valve positioned at least partially within the handle; and
a loader assembly comprising a loader coupler and a loader tubing, the loader tubing extending through a proximal end of the handle through the hemostasis valve.

15. The delivery device of claim 14, wherein the loader tubing extends distal to the hemostasis valve and at least 3 inches through the catheter sheath.
